**Europäisches Patentamt**

**European Patent Office**

**Office européen des brevets**

(19)

(11) Numéro de publication: **0 085 604**
**B1**

(12) ## FASCICULE DE BREVET EUROPEEN

(45) Date de publication du fascicule du brevet:
**06.11.85**

(51) Int. Cl.⁴: **C 07 C 102/00**, C 07 C 103/90

(21) Numéro de dépôt: **83400152.1**

(22) Date de dépôt: **21.01.83**

(54) Procédé amélioré de fabrication de l'alpha-acétoxy alpha-méthyl N,N'-diacétyl malonamide de haute productivité.

(30) Priorité: **02.02.82 FR 8201624**

(43) Date de publication de la demande:
**10.08.83 Bulletin 83/32**

(45) Mention de la délivrance du brevet:
**06.11.85 Bulletin 85/45**

(84) Etats contractants désignés:
**BE CH DE FR GB IT LI LU NL SE**

(56) Documents cités:
**EP - A - 0 019 227**
**FR - A - 2 165 631**
**FR - A - 2 363 541**

(73) Titulaire: **ATOCHEM, 12/16, allée des Vosges,**
**F-92400 Courbevoie (FR)**

(72) Inventeur: **Dubreux, Bernard, "Le Grillon" Tour E**
**Chemin de champgrillet, F-69340 Francheville-Le-Bas**
**(FR)**
Inventeur: **Laviron, Charles, 27 A, rue Soeur Bouvier,**
**F-69005 Lyon (FR)**

(74) Mandataire: **Rochet, Michel et al, ATOCHEM**
**Département Propriété Industrielle Cédex 22,**
**F-92091 Paris la Défense (FR)**

## Description

La présente invention concerne une amélioration du procédé de fabrication de l'α-acétoxy, α-méthyl--N,N'-diacétylmalonamide décrit dans le brevet français n° 2 363 541.

L'α-acétoxy, α-méthyl-N,N'-diacétylmalonamide est un activateur de persels trouvant une application dans des compositions de blanchiment ou de récurage actives ainsi à plus basse température. Ce composé et un mode de préparation ont été décrits dans le brevet français n° 2 363 541. Une façon particulièrement avantageuse d'opérer consiste d'après ce brevet à faire réagir dans une première étape des cyanures ou de l'acide cyanhydrique et un catalyseur basique sur de l'anhydride acétique, puis de traiter le mélange ainsi obtenu par de l'acide acétique en présence d'un catalyseur acide.

L'objet de la présente invention est d'améliorer la productivité de la première étape de ce procédé.

La demanderesse a découvert que l'on pouvait réaliser plus économiquement la synthèse de l'α--acétoxy, α-méthyl-N,N'-diacétylmalonamide en augmentant la productivité de la première étape en effectuant cette première étape en continu et en présence de catalyseurs à base de résines échangeuses d'anions.

La présente invention fournit donc un procédé de préparation de l'α-acétoxy-α-méthyl-N,N'-diacétyl-malonamide dans lequel on fait réagir, dans une première étape, un composé choisi parmi l'acide cyanhydrique et les cyanures sur de l'anhydride acétique, et, dans une deuxième étape, on traite le mélange ainsi obtenu par de l'acide acétique en présence d'un catalyseur acide, caractérisé par le fait que l'on effectue la première étape en présence d'un catalyseur à base de résines échangeuses d'anions dont les groupements fonctionnels sont choisis parmi les groupements ammonium quaternaire et les groupements amine tertiaire.

Les catalyseurs utilisables selon l'invention sont toutes les résines échangeuses d'anions dont les groupements fonctionnels sont des ammoniums quaternaires ou des amines tertiaires, comme par exemple des groupes benzyldiméthylamine ou benzyltriméthylammonium portés par des polymères réticulés comme par exemple des copolymères styrène divinylbenzène.

Pour la mise en œuvre du procédé on injecte une solution d'acide cyanhydrique dans l'anhydride acétique dans un réacteur tubulaire contenant la résine imprégnée d'acide acétique, et dont les groupements fonctionnels sont sous forme d'acétate d'ammonium tertiaire ou quaternaire.

Le rapport molaire anhydride acétique/acide cyanhydrique peut varier dans de larges proportions, mais, pour des raisons évidentes de productivité, reste préférablement compris entre 1 et 1,2 environ.

La température de la réaction est comprise entre 20 et 100°C, de préférence entre 60° et 90°C environ.

Les exemples suivants illustrent l'invention de façon non limitative.

## Exemples 1 et 2

Le réacteur mis en œuvre est constitué d'un tube de 1 200 mm de longueur et de 10 mm de diamètre enroulé en spirale de 80 mm de diamètre moyen, entièrement rempli de résine échangeuse d'ion sous forme d'acétate d'ammonium, et plongé dans un bain thermostaté. On introduit dans ce réacteur à l'aide d'une pompe un mélange homogène constitué de 19,3% d'acide cyanhydrique et de 80,7% d'anhydride acétique. Après le temps nécessaire à la mise en régime, 100 g de la liqueur sortant du réacteur sont prélevés et coulés en l'espace de 30 minutes dans un réacteur agité maintenu à 50°C, contenant 30 g d'acide acétique glacial et 7,5 ml d'acide sulfurique à 70° Baumé. Après 2 heures à 50°C le précipité d'α-acétoxy, α-méthyl-N,N'-diacétylmalonamide est filtré, lavé avec 100 ml d'eau et séché.

Dans le tableau I ci-après figurent les rendements en produit final comptés par rapport à l'acide cyanhydrique, et les quantités de produit final potentiel formé à la fin de la première étape rapportées à l'heure de temps de séjour et au litre de réacteur selon la présente invention et selon le brevet français 2 363 541.

| | Exemple 1 | Exemple 2 | Brevet français n° 2 363 541 (exemple 2) |
|---|---|---|---|
| Catalyseur | Lewatit résine MP 500 (Bayer) | Lewatit résine MP 62 (Bayer) | Triéthyl-amine |
| Température | 60°C | 60°C | 50°C |
| Rendement sur HCN | 85% | 86% | 84% |
| Quantité de produit final pour 1 heure de temps de séjour et 1 litre de réacteur | 532 g | 540 g | 87 g |

## Revendication

Procédé de préparation de l'α-acétoxy-α-méthyl--N,N'-diacétylmalonamide dans lequel on fait réagir, dans une première étape, un composé choisi parmi l'acide cyanhydrique et les cyanures sur de l'anhydride acétique et, dans une deuxième étape, on traite le mélange ainsi obtenu par de l'acide acétique en présence d'un catalyseur acide, caractérisé par le fait que l'on effectue la première étape en présence d'un catalyseur à base de résines échangeuses d'anions dont les groupements fonctionnels sont choisis parmi les groupements ammonium quaternaires et les groupements amine tertiaire.

**Patentanspruch**

Verfahren zur Herstellung von α-Acetoxy-α-methyl-N,N'-diacetylmalonamid, in dem man in einer ersten Stufe eine Verbindung, die unter der Cyanwasserstoffsäure und den Cyaniden ausgewählt ist, mit Acetanhydrid umsetzt und in einer zweiten Stufe die so erhaltene Mischung mit Essigsäure in Gegenwart eines sauren Katalysators behandelt, dadurch gekennzeichnet, dass man die erste Stufe in Gegenwart eines Katalysators auf Basis von Anionenaustauscherharzen durchführt, deren funktionelle Gruppen unter den quartären Ammonium- und den tertiär-Amingruppen ausgewählt sind.

**Claim**

Process for the preparation of α-acetoxy-α-methyl-N,N'-diacetylmalonamide in which, in a first stage, a compound chosen from among hydrocyanic acid and cyanides is reacted with acetic anhydride and, in a second stage, the mixture thus obtained is treated with acetic acid in the presence of an acid catalyst, characterised in that the first stage is carried out in the presence of a catalyst based on anion resins, the functional groups of which are chosen from among quaternary ammonium groups and tertiary amine groups.